# EUROPÄISCHE PATENTSCHRIFT

(11) **EP 0 980 675 B1**
(45) Veröffentlichungstag und Bekanntmachung des Hinweises auf die Patenterteilung: **04.02.2004**
(21) Anmeldenummer: 99115965.8
(22) Anmeldetag: 13.08.1999
(51) Int. Cl.: A61B 17/115

(54) **Vorrichtung zum Anschliessen eines chirurgischen Nahtklammergeräts an eine Bedienvorrichtung**
Device connecting a surgical stapler to an actuator
Dispositif de liaison d'une agrafeuse chirurgicale à un dispositif d'actionnement

(30) Priorität: 17.08.1998 DE 19836950
(43) Veröffentlichungstag der Anmeldung: 23.02.2000
(73) Patentinhaber: Deutsches Zentrum für Luft- und Raumfahrt e.V., 53175 Bonn (DE)
(72) Erfinder: Balazs, Mathias, 82284 Grafrath (DE); Hagn, Ulrich, 82234 Wessling (DE)
(74) Vertreter: von Kirschbaum, Albrecht, Dipl.-Ing.

(56) Entgegenhaltungen:
- EP-A- 0 336 596
- EP-A- 0 481 619
- WO-A-98/27873
- DE-A- 4 327 233
- DE-A- 19 509 115
- US-A- 5 391 166
- US-A- 5 609 285

## Beschreibung

Die Erfindung betrifft ein chirurgisches Instrument in Form eines Nahtklammergeräts, das einen Skalpellträger, ein Zirkularskalpell, ein Klammermagazin mit Klammern und eine den Klammern zugeordnete Ausstoßvorrichtung sowie einen Andrückkörper als Funktionselemente aufweist, welche mittels einer vom chirurgischen Element lösbaren Bedienvorrichtung mit Griff- und Schaftteil betätigbar sind.

In US 4,573,468 ist ein zirkulares Nahtklammergerät beschrieben, bei welchem die komplette Kopfeinheit abgenommen werden kann. Der Anschluß der Kopfeinheit an ein Bedienteil erfolgt bei diesem Nahtklammergerät über einen aufwendigen Bajonettverschluß. Ferner muß bei dem bekannten Gerät der Andruckkörper an- bzw. abgeschraubt werden, was umständlich und zeitaufwendig ist.

In US 5,533,361 ist ein Kopfteil eines chirurgisches Instruments mit einer formschlüssigen Verbindung durch ein gequetschtes Rohr steif und untrennbar von einem Schafteil der Bedienvorrichtung verbunden. In US 4,606,343 sind ein Schaftrohr der Bedieneinrichtung und ein Kopfteil des chirurgischen Instruments fest miteinander verbunden. Somit ist ein Auswechseln von Funktions-Teilen dieses chirurgischen Instruments nicht vorgesehen.

In US 5,391,166 sind endoskopische chirurgische Instrumente beschrieben, die von einem wiederverwendbaren Griffteil abnehmbare Bearbeitungsenden aufweisen. Jedes abnehmbare Bearbeitungsende weist bipolare Elektroden an einander gegenüberliegenden beweglichen Teilen auf, um einen hochfrequenten Strom durchzuleiten, um gleichzeitig Gewebe durchzutrennen und um eine Blutstillung bezüglich des Gewebes durchzuführen. Zum Auswechseln der Bearbeitungsabschnitte muss eine ausreichende Kraft auf abnehmbare Bearbeitungsabschnitte aufgebracht werden, während gleichzeitig ein langgestreckter mit dem Griffteil verbundener Schaftteil gehalten werden muss.

Aufgabe der Erfindung ist es, ein chirurgisches Instrument in Form eines Nahtklammergeräts so auszubilden, dass deren Funktionselemente schnell ausgewechselt werden können und eine einwandfreie Bedienbarkeit des Instruments erhalten bleibt. Gemäß der Erfindung ist diese Aufgabe bei einem chirurgischen Instrument in Form eines Nahtklammergeräts durch die Merkmale im kennzeichnenden Teil des Anspruchs 1 gelöst. Weiterbildungen der Erfindung sind Gegenstand der auf den Anspruchs 1 unmittelbar oder mittelbar rückbezogenen Ansprüche.

Gemäß der Erfindung ist zum lösbaren Anschließen des chirurgischen Elements in Form eines Nahtklammergeräts am Schaftteil einer Bedienvorrichtung am proximalen Ende des Instruments ein Druckknopfanschluss mit an Biegelaschen sitzenden Druckknöpfen ausgebildet, während am distalen Ende des Schaftteils zum Eingreifen der Druckknöpfe eine zu diesen korrespondierende Aufnahme vorgesehen ist. Zum lösbaren Anschließen von lösbaren Funktionselementen des Instruments an dessen proximalen Ende ist ein Verbindungsteil vorgesehen, das mit einem am distalen Ende des Schaftteils ausgebildeten Anschluss mittels eines im Schaftteil untergebrachten von der Bedienungsvorrichtung aus betätigten Verstellmechanik verriegelbar ausgebildet ist. Ferner sind gemäß der Erfindung zum gleichzeitigen Durchführen einer Klammerung und eines Gewebeschnitts die Funktionselemente Skalpellträger und Klammerausstoßvorrichtung als festgekoppelte Einheit axial verschiebbar.

Gemäß einer vorteilhaften Variante der Erfindung sind dieselben Funktionselemente, nämlich Skalpellträger und Klammerausstoßvorrichtung axial verschiebbar in der Weise gekoppelt, dass nach Durchführen einer Klammerung der Skalpellträger und die Klammerausstoßvorrichtung entkoppelt werden und anschließend durch Verfahren des Skalpellträgers mittels des Skalpells ein Gewebeschnitt durchgeführt wird.

Hierbei eignen sich als Kopplungs-/Entkopplungselement zwischen Skalpellträger und Klammerausstoßvorrichtung entweder ein biegeelastisches Teil oder eine Grenzkraftsperre, die bei Überschreiten der Grenzkraft die Funktionselemente voneinander entkoppelt. Eine derartige Grenzkraftsperre kann als Sollbruchelement, als Blattfederraste oder auch beispielsweise als Kugelfederraste ausgeführt sein. Zum Koppeln/Entkoppeln einer wellenförmigen Innenstruktur ist an der Klammerausstoßvorrichtung eine korrespondierende Außenkontur des Skalpellträgers mittels Keilwirkung zugeordnet.

Ferner weist gemäß der Erfindung das Verbindungsteil, das ein rohrförmiger, am proximalen Ende geschlitzter Stiftkörper ist, Verzahnungsbereiche auf und ist auf einen im Schaftteil untergebrachten Anschluss der von der Bedienungseinrichtung aus betätigten Verstellmechanik aufsteckbar und verriegelbar.

Gemäß der Erfindung lassen sich somit schnell und intuitiv ein chirurgisches Instrument in Form eines Nahtklammergeräts an den Schaftteil einer Bedienungseinrichtung und gleichzeitig wichtige Funktionselemente des Nahtklammergeräts über ein am distalen Ende des Schaftteils der Bedieneinrichtung angebrachtes Verbindungsteil anschließen.

Nachfolgend wird die Erfindung an Hand von bevorzugten Ausführungsformen unter Bezugnahme auf die Zeichnungen im Einzelnen Erläutert. Es zeigen:
- Fig.1: in perspektivischer Darstellung drei Hauptbaugruppen einer Kopfeinheit;
- Fig.2a bis 2c: ebenfalls in perspektivischer Darstellung jeweils Explosionsansichten der Einzelteile der drei Hauptbaugruppen der Kopfeinheit von Fig.1, und zwar in Fig.2a diejenigen der Baugruppe Klammermagazin, in Fig.2b diejenigen der Baugruppe Verbindungsstift und in Fig.2c diejenigen der Baugruppe Andruckkörper;
- Fig.3: eine Schnittansicht der an einem Schaft montierten Kopfeinheit mit ausgefahrenem Andruckkörper;
- Fig.4: ebenfalls im Schnitt eine Darstellung der an einem Schaft montierten Kopfeinheit der Fig.3 mit zurückgefahrenem Andruckkörper und durchgeführter Klammerung;
- Fig.5: eine Schnittansicht durch den Andruckkörper entlang einer Linie 5-5 in Fig.4;
- Fig.6: eine vergrößerte perspektivische Darstellung eines am distalen Ende eines Schaftteils angebrachten Druckknopf-Anschlusses mit einem diesen zugeordneten Knopfmechanismus am proximalen Ende einer Kopfeinheit;
- Fig.7: eine perspektivische Darstellung eines Abschnitts am distalen Ende eines Schlußstiftes und einer Aufnahme an einem Verbindungsstift;
- Fig.7a: stark vergrößert ein Detail des Anschlußstiftes
- Fig.8: eine modifizierte Ausführung des distalen Endes eines Schaftteils und ein diesem angepaßtes proximales Ende einer Kopfeinheit;
- Fig.9a und 9b: im Schnitt eine Teilansicht eines Skalpellträgers und eines damit festgekoppelten Klammerausstoßers, und zwar in Fig.9a in zurückgezogener Stellung und in Fig.9b in ausgefahrener Stellung;
- Fig.10a und 10b: im Schnitt eine Teilansicht einer ersten Ausführungsform eines Skalpellträgers und eines mit diesem über ein Kopplungselement verbundenen Klammerausstoßers, und zwar in Fig.10a in zurückgefahrener Stellung und in Fig.10b in ausgefahrener Stellung;
- Fig.11a und 11b: im Schnitt eine Teilansicht einer zweiten Ausführungsform eines Skalpellträgers und eines mit diesem über ein Kopplungselement verbundenen Klammerausstoßers, und zwar in Fig.11a in zurückgefahrener Stellung und in Fig.11b in ausgefahrener Stellung;
- Fig.12a und 12b: im Schnitt eine Teilansicht einer dritten Ausführungsform eines Skalpellträgers und eines mit diesem über ein Kopplungselement verbundenen Klammerausstoßers, und zwar in Fig. 12a in zurückgefahrener Stellung und in Fig.12b in ausgefahrener Stellung;
- Fig.13a und 13b: im Schnitt eine Teilansicht einer vierten Ausführungsform eines Skalpellträgers und eines mit diesem über ein Kopplungselement verbundenen Klammerausstoßers, und zwar in Fig.13a in zurückgefahrener Stellung und in Fig.13b in ausgefahrener Stellung und
- Fig.14a und 14b: im Schnitt eine Teilansicht einer fünften Ausführungsform eines Skalpellträgers und eines mit diesem über ein Kopplungselement verbundenen Klammerausstoßers, und zwar in Fig.14a in zurückgefahrenen Stellung und in Fig.14b in ausgefahrener Stellung.

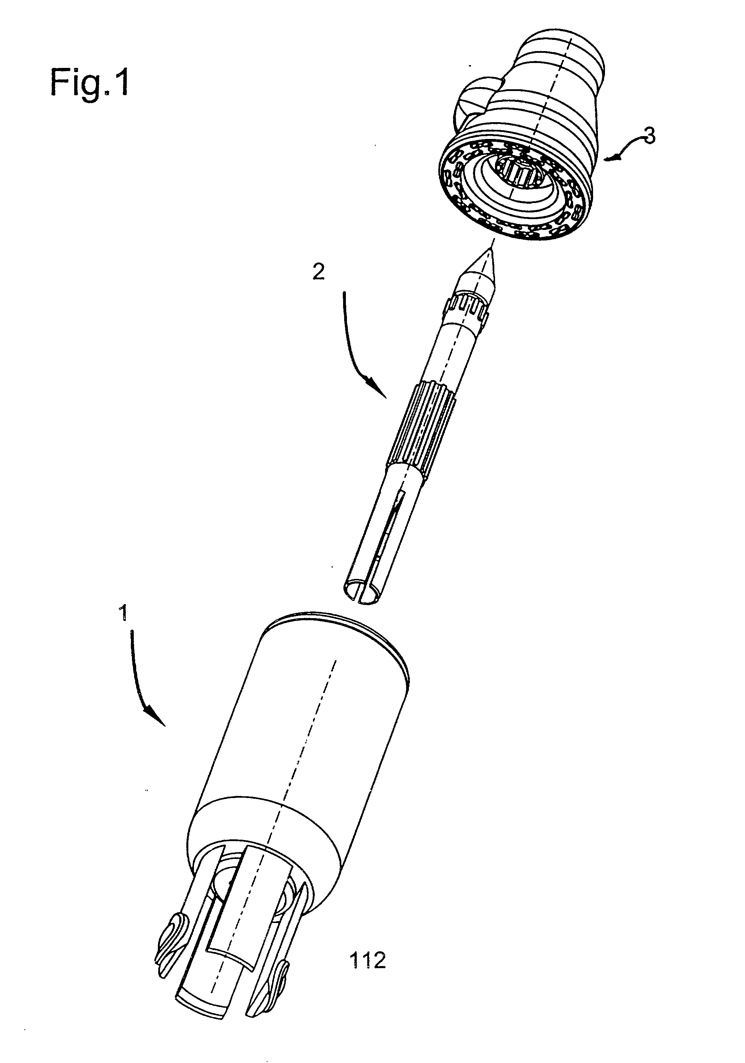

Fig.1 zeigt die Aufteilung einer Kopfeinheit eines zirkularen Nahtklammergerätes in drei Baugruppen, nämlich ein Klammermagazin 1, einen Verbindungsstift 2 und einen Andruckkörper 3.

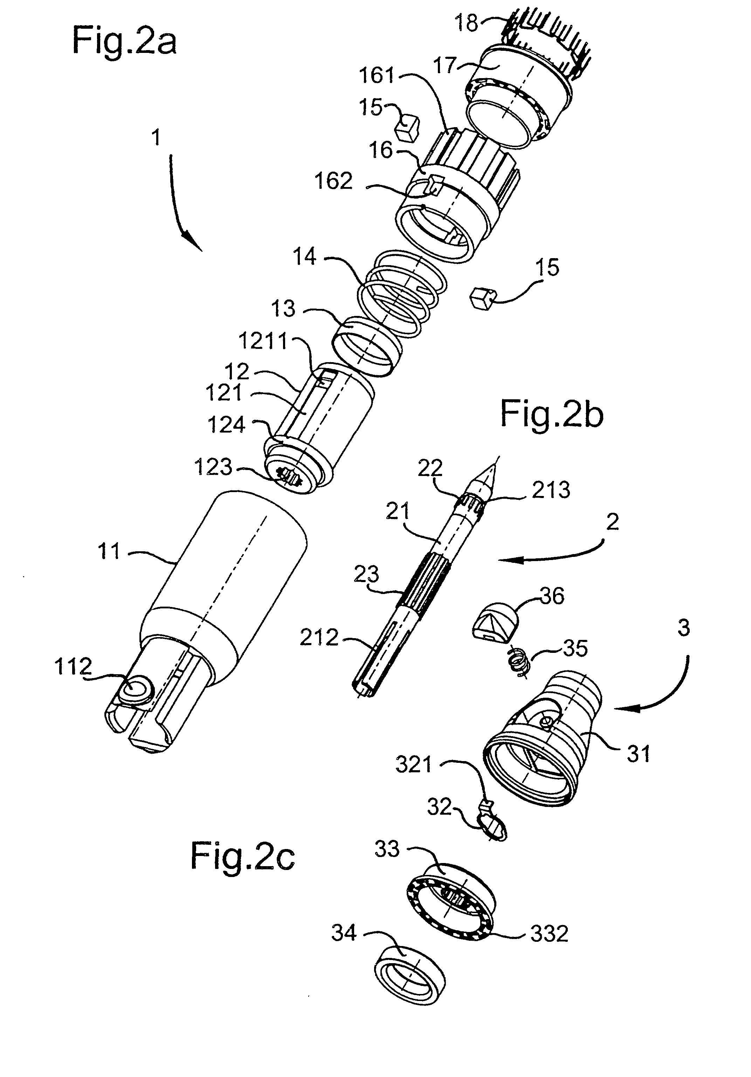

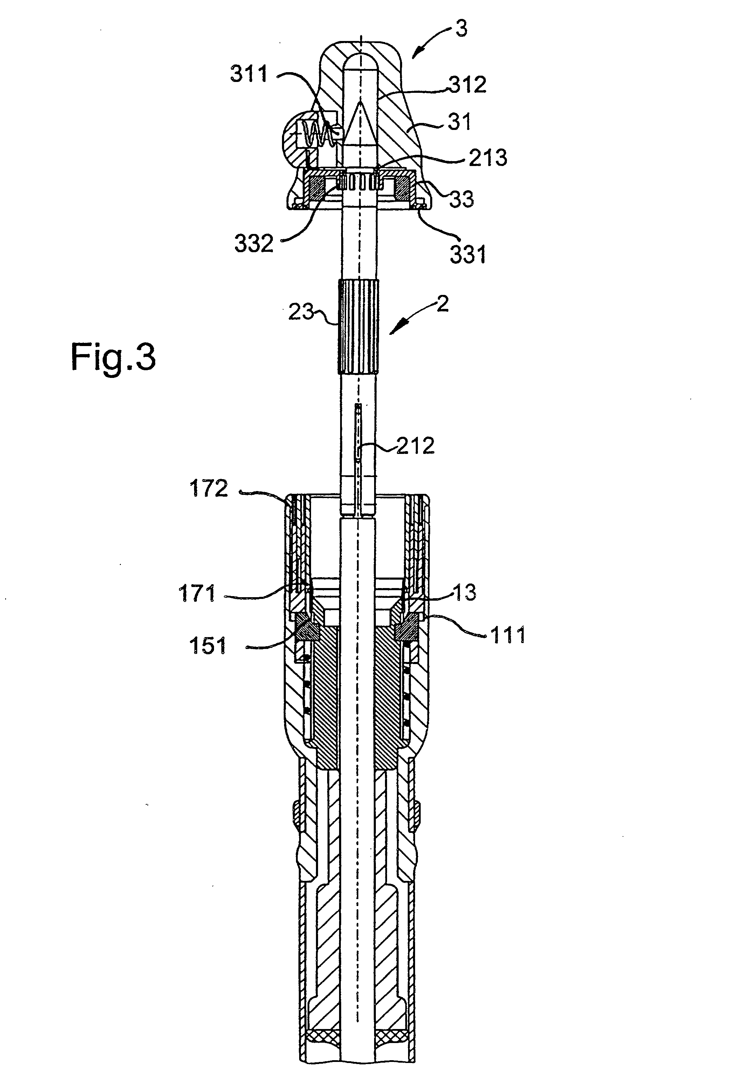

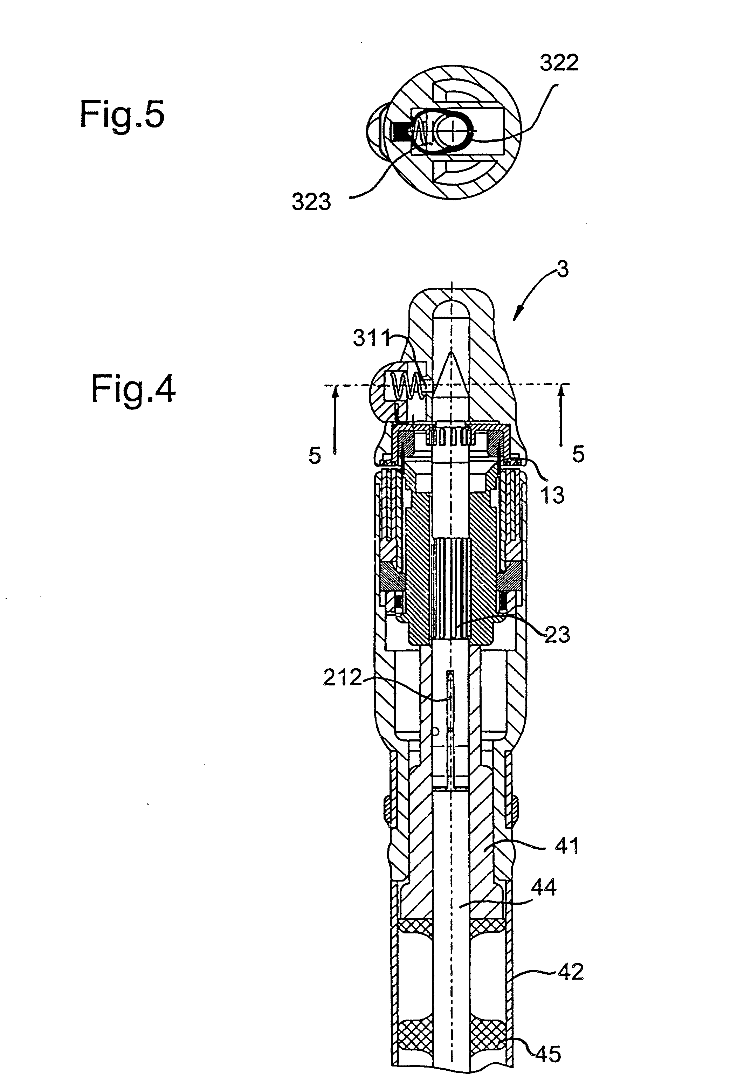

Wie in Fig.1 und Fig.2a dargestellt, ist das Klammermagazin 1 in einem Gehäuse 11 einer Kopfeinheit des zirkularen Nahtklammergerätes untergebracht, welches über einen Druckknopfanschluß 112 mit einem Schaftrohr 42 (Fig.4) des Nahtklammergerätes verbunden ist. In einem fest mit dem Gehäuse 11 verbundenen Klammermagazin 17 sind in mindestens einem Klammerschacht 172 (Fig.3) u-förmige Klammern 18 konzentrisch angeordnet. Zu dem oder den Klammerschächten 172 sind Stempel 161 eines im Gehäuse 11 axial verschiebbar gelagerten Klammerausstoßers 16 ausgerichtet.

Im Gehäuse 11 der Kopfeinheit ist ferner axial verschiebbar ein Skalpellträger 12 mit aufgebrachtem Skalpell 13 gelagert. Skalpellträger 12 und Klammerausstoßer 16 sind über Kopplungselemente 15 miteinander verbunden. Mittels einer Feder 14, die den Skalpellträger 12 an einem Absatz 124 gegen den Klammerausstoßer 16 abstützt, wird der Skalpellträger 12 mit dem Skalpell 13, nachdem eine Klammerung und vorzugsweise ein Gewebeschnitt durchgeführt sind, wieder in das Gehäuse 11 zurückgefahren.

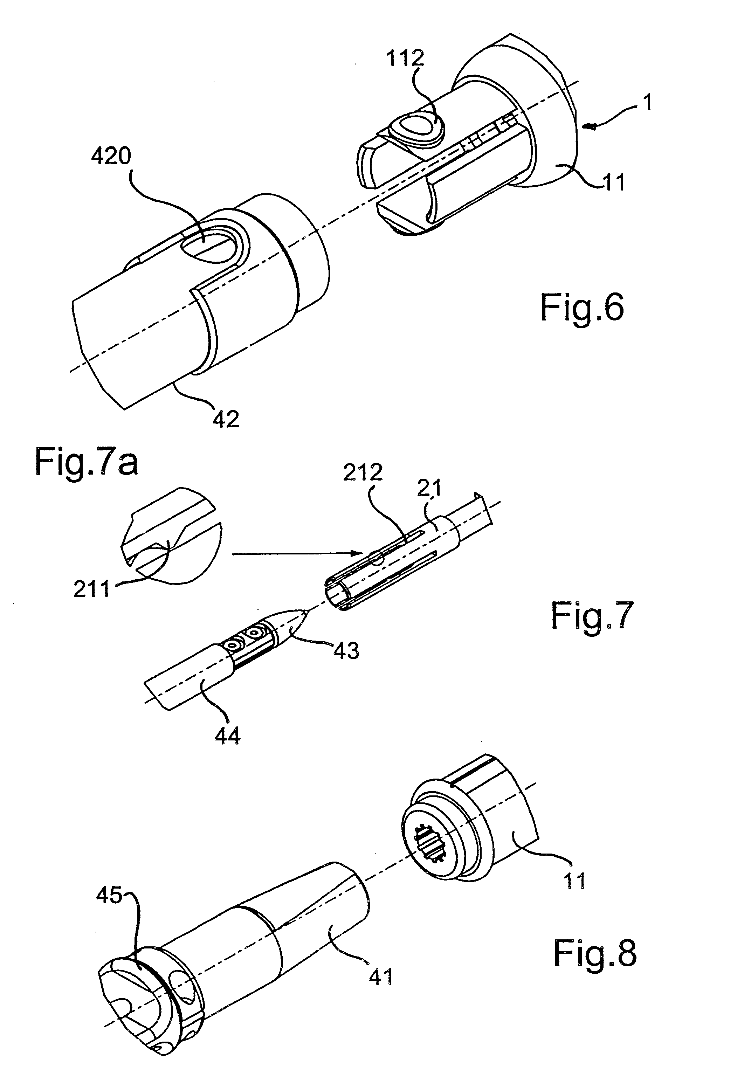

Wie in Fig.2b dargestellt, hat ein Stiftkörper 21 des Verbindungsstiftes 2 ein rohrförmiges, geschlitztes Ende 212, das auf einen Anschlußstift 43 einer Andruckkörper-Verstellmechanik 44 (siehe Fig.7) des Nahtklammergeräts aufsteckbar ist. Dabei greift eine umlaufende Nut des Anschlußstiftes 43 in einen umlaufenden Absatz 211 (Fig.7a) der Innenkontur des Stiftes 21 ein.

Beim Einstellen eines Gewebespaltes wird mittels der Andruckkörper-Verstellmechanik 44 des Nahtklammergerätes der Anschlußstift 43 mit dem Verbindungsstift 2 in das Innere des Schaftrohres 42 hineingezogen (siehe Fig.4). Dadurch kommt das geschlitzte Ende 212 des Verbindungsstiftes 2 innerhalb eines ballig ausgeführten Druckteils 41 des Nahtklammergerätes zu liegen. Dadurch ist die Verbindung des Anschlußstiftes 43 mit dem Verbindungsstift 2 arretiert.

Verzahnungen 23 bzw. 22 richten den Verbindungsstift 2 rotatorisch zu Verzahnungen 123 des Skalpellträgers 12 bzw. zu Verzahnungen 332 einer Klammermatrize 33 (Fig.2c) aus. Dadurch ist ein exaktes Ausrichten der Klammerschächte 172 des Magazins 17 zu Klammerumformnuten 331 der Matrize 33 gewährleistet. (Siehe Fig.3 oben).

Am distalen Ende des Verbindungsstiftes 2 ist der Andruckkörper 3 lösbar angeschlossen. Dabei greift der kleinere Innendurchmesser 322 (Fig.5) eines Riegelelements 32 in eine korrespondierende Nut 213 des Verbindungsstiftes 2 (Fig.2b) ein. Zum Lösen der Verbindung zwischen dem Verbindungsstift 2 und dem Andruckkörper 3 muß ein Bedienknopf 36 gegen die Federkraft einer Feder 35 betätigt werden, die sich auf einem Dorn 311 einer Hutkappe 31 zentriert. Dabei wird das mit einer Nase 321 in den Bedienknopf 36 eingehängte Riegelelement 32 so verschoben, daß der größere Durchmesser 323 (Fig.5) des Riegelelements 32 zu der Längsachse des Verbindungsstiftes 2 ausgerichtet ist.

Der Durchmesser 323 ist etwas größer bemessen als der Außendurchmesser des Verbindungsstift 2 oder einer Führungsbohrung 312 (Fig.3, oben), so daß dieser sich in dieser Stellung aus dem Andruckkörper 3 herausziehen läßt. Der Andruckkörper 3 weist die Klammerumformnuten 331 zum Umformen der Klammern 18 sowie einen vorzugsweise aus Teflon hergestellten Ring 34 auf, der als Schneiden-Gegenlager für das Skalpell 13 dient. Während des Klammerungsvorganges wird somit eine Axialkraft vom ballig ausgeführten Druckteil 41 auf den Skalpellträger 12 ausgeübt.

In Fig.6 ist in einer perspektivischen Darstellung ein Druckknopfanschluß 112 wiedergegeben, der am proximalen Ende des Gehäuses 11 der Kopfeinheit vorgesehen ist und an Biegelaschen sitzende Druckknöpfe aufweist, die in korrespondierende Radialbohrungen 420 des Schaftrohrs 42 eingreifen.

In Fig.7 ist in perspektivischer Darstellung das geschlitzte Ende 212 des Stiftkörpers 21 dargestellt. Wie in der vergrößerten Detaildarstellung in Fig.7a wiedergegeben ist, greift ein umlaufender Absatz 211 der Innenkontur des geschlitzten rohrförmigen Endes 212 in eine korrespondierende Nut am Anschlußstift 43 einer nicht näher dargestellten Andruckkörper-Verstellmechanik 44 eines Bedienteils ein.

In Fig.8 ist ein Teil einer Klammerungsmechanik 45 des Bedienteils wiedergegeben, die in ein ballig ausgeführtes Druckteil 41 übergeht, durch das eine Bedienkraft als axiale Druckkraft in den Körper einer Kopfeinheit weitergeleitet wird.

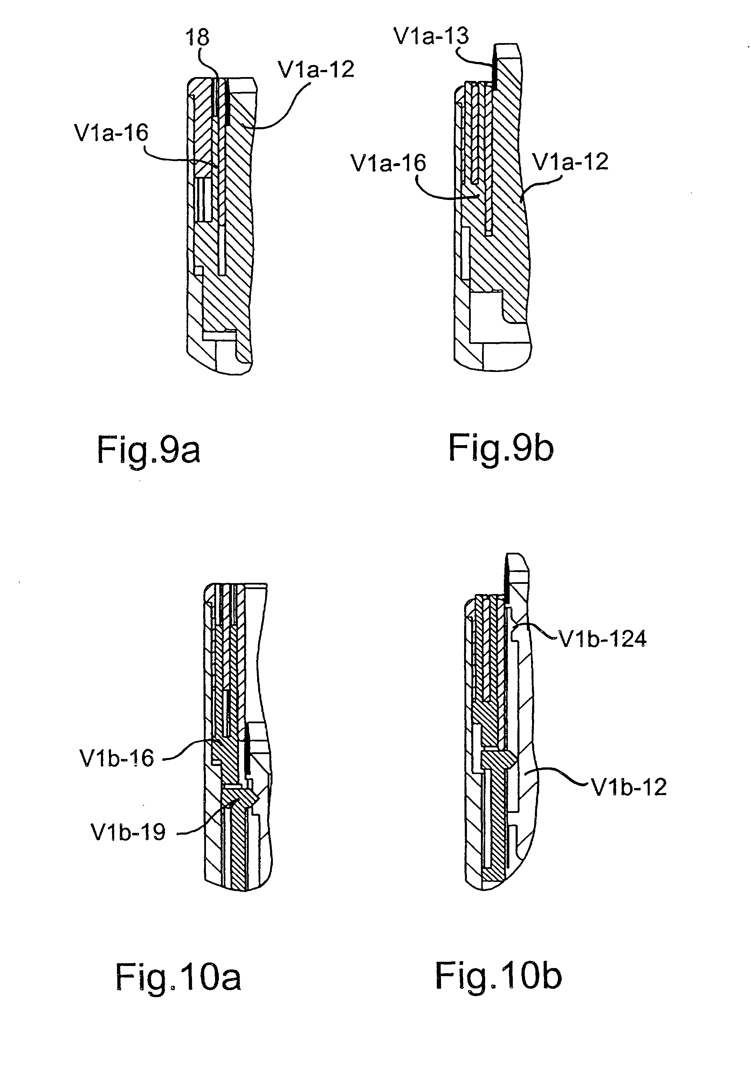

In einer ersten. in Fig.9a und 9b dargestellten Ausführungsform, die als Variante 1a bezeichnet ist, sind Skalpellträger V1a-12 und Klammerausstoßer V1a-16 festgekoppelt, d.h. Klammerung und Gewebeschnitt finden gleichzeitig statt. Bei einem Klammerungsvorgang wird die Einheit aus Skalpellträger V1a-12 und Klammerausstoßer V1a-16 axial in dem Gehäuse 11 der Kopfeinheit verschoben (Fig. 9b). Dadurch werden die Klammern 18 aus ihren Schächten ausgeschoben und in den Klammerumformnuten 331 der Matrize 33 umgebogen. Zeitgleich schneidet ein Zirkularskalpell V1a-13 überstehenden Geweberand ab.

Bei allen nachfolgend beschriebenen Ausführungsformen findet eine Klammerung vor dem Gewebeschnitt statt. Bei der in Fig.2a bis Fig.4 sowie Fig.10a und 10b dargestellten, diesbezüglich modifizierten Ausführungsform, die daher als Variante V1b bezeichnet ist und bei der es sich um eine wegsensitive Kopplung handelt, ist der Klammerausstoßer V1b-16 über mindestens ein Kopplungselement 15 (Fig.2a) mit dem Skalpellträger V1b-12 verbunden. Das mindestens eine Kopplungselement 15 sitzt in einem Fenster 162 des Klammerausstoßers 16 sowie in einer Vertiefung 1211 einer Führungsnut 121 des Skalpellträgers 12 (Fig.2a). Zu Beginn einer Klammerung werden Klammerausstoßer V1b-16 und Skalpellträger V1b-12 gemeinsam bewegt (Fig.3).

Nach einem bestimmten, zurückgelegtem Klammerungsweg läßt es die Ausbildung einer Innenkontur 111 des Gehäuses 11 zu, daß die Kopplungselemente 15 radial verschoben werden können. Dadurch trifft eine Keilfläche 151 (Fig.3) jedes Kopplungselementes 15 auf einen Absatz 171 des Klammermagazins 17, wodurch das Kopplungselement 15 radial nach außen verschoben wird, bis es nicht mehr mit dem Skalpellträger 12 in Eingriff steht. In der Folge sind daher Klammerausstoßer 16 und Skalpellträger 12 entkoppelt. Anschließend wird der Skalpellträger 12 allein verfahren und führt den Gewebeschnitt aus. (Siehe Fig. 4).

Bei der in Fig.10a und Fig.10b dargestellten ersten Ausführungsform Variante V1b ist der Skalpellträger V1b-12 mit Klammerausstosser V1b-16 über mindestens eine biegebalkenartige Lasche V1-19 verbunden. Diese Lasche V1b-19 greift in eine korrespondierende Vertiefung V1b-124 des Skalpellträgers V1b-12 ein und überträgt während des Klammerungsvorganges die Kraft vom Skalpellträger V1b-12 auf den Klammerausstosser V1b-16 (siehe Fig.10a).

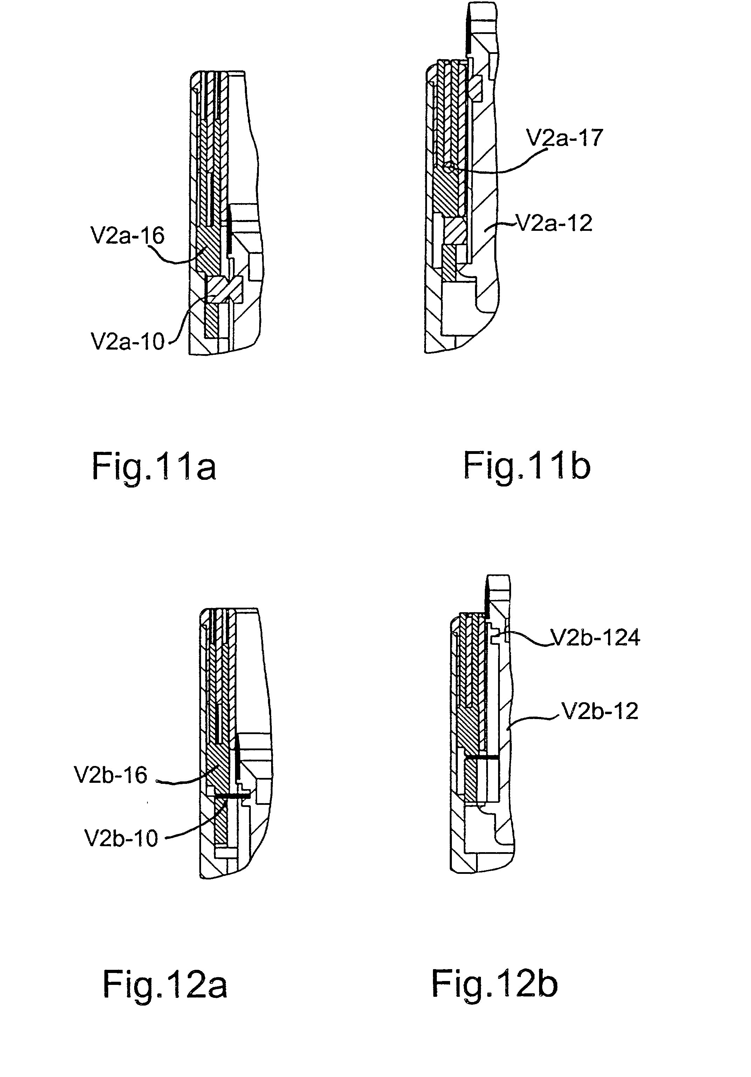

Ist die Klammerung durchgeführt, stößt der Klammerausstosser V1b-16 am Klammermagazin an; die Laschen V1b-19 befinden sich nun in einem Bereich der Innenkontur 111 des Gehäuses 11 (Fig.3), in dem die Laschen V1b-19 radial nach außen gebogen werden können. Durch keilförmige Flächen der Vertiefung V1b-124 werden die Laschen V1b-19 radial nach außen gedrückt, wodurch der Skalpellträger V1b-12 vom Klammerausstoßer V1b-16 entkoppelt wird. Der Skalpellträger V1b-12 wird anschließend allein verfahren und führt den Gewebeschnitt durch. (Siehe Fig. 10b) Bei den nachstehend anhand der Fig.11a/11b, Fig. 12a/12b, Fig.13a/13b sowie Fig.14a/14b beschriebenen Ausführungsformen, die als Varianten V2a bis V2d bezeichnet sind, findet dagegen eine sogenannte kraftsensitive Kopplung statt.

Die Klammerung findet auch bei diesen Ausführungsformen vor dem Gewebeschnitt statt. Klammerausstoßer V2a-16, V2b-16, V2c-16 bzw. V2d-16 sowie Skalpellträger V2a-12, V2b-12, V2c-12 bzw. V2d-12 sind über mindestens eine Grenzkraftsperre V2a-10, V2b-10, V2c-10 bzw. V2d-160 miteinander gekoppelt. Während der Klammerung verfahren die Klammerausstoßer V2a-16, V2b-16, V2c-16 bzw. V2d-16 und die Skalpellträger V2a-12, V2b-12, V2c-12 bzw. V2d-12 gemeinsam.

Nachdem die Klammerung durchgeführt ist, stößt der Klammerausstoßer V2a-16, V2b-16, V2c-16 bzw. v2d-16 an das Klammermagazin, beispielsweise V2a-17 in Fig.llb. Überschreitet die Klammerungskraft die Grenzkraft der jeweiligen Grenzkraftsperre V2a-10, V2b-10 bzw. V2c-10, so entkoppeln sich Klammerausstoßer V2a-16, V2b-16, V2c3-16 bzw. V2d-16 und Skalpellträger V2a-12, V2b-12, V2c-12 bzw. V2d-12, so daß letzterer anschließend allein verfährt und den Gewebeschnitt ausführt.

In der in Fig.11a und 11b dargestellten zweiten Ausführungsform (Variante V2a) ist die Grenzkraftsperre V2a-10 durch ein Sollbruchelement realisiert. Bei Überschreiten der Grenzkraft bricht das Sollbruchelement, so daß daraufhin der Skalpellträger V2a-12 und Klammerausstoßer V2a-16 entkoppelt sind.

Die Grenzkraftsperre V2b-10 der in Fig. 12a und 12b dargestellten Variante V2b ist durch eine Blattfederraste V2b-10 realisiert, die an einer Vertiefung V2b-124 des Skalpellträgers V2b-12 aufsitzt. Bei ausreichend hoher Grenzkraft verformt sich die Blattfederraste V2b-10 wie ein Biegebalken, wodurch sie aus der Vertiefung V2b-124 schnappt. Dadurch sind Skalpellträger V2b-12 und Klammerausstoßer V2b-16 voneinander entkoppelt.

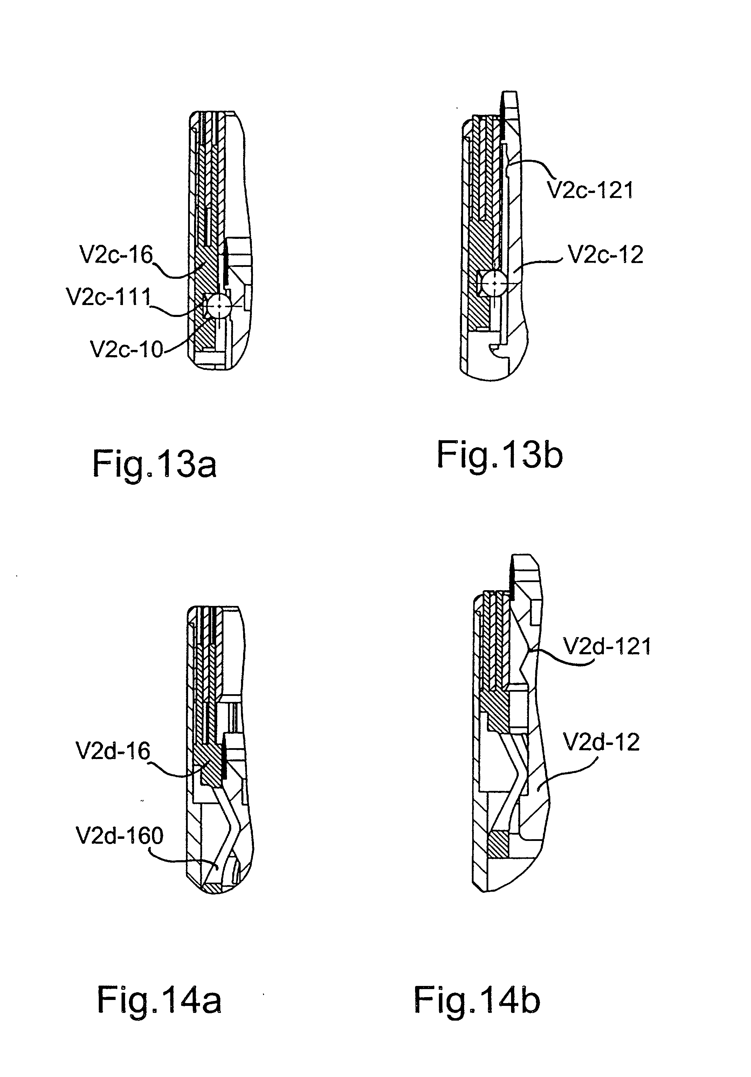

In der in Fig.13a und 13b dargestellten dritten Ausführungsform (Variante V2c) ist die Grenzkraftsperre eine Kugelfederraste. Eine Kugel V2c-10 wird durch eine Feder V2c-111 in eine Vertiefung V2c-121 des Skalpellträgers V2c-12 gedrückt. Ist die Grenzkraft beim Anschlag des Klammerausstoßers V2c-16 groß genug, so schnappt die Kugel V2c-10 aus der Vertiefung V2c-121 heraus. Hierdurch wird der Klammerausstoßer V2c-16 von dem Skalpellträger V2c-12 entkoppelt.

Bei der in Fig.14a und 14b dargestellten vierten Ausführungsform (Variante V2d) weist das Kopplungselement eine wellenförmige Innenkontur V2d-160 des Klammerausstoßers V2d-16 auf, welche in eine korrespondierende Außenkontur V2d-121 des Skalpellträgers V2d-12 eingreift. Sollen nach der Klammerung der Klammerausstoßer V2d-16 und der Skalpellträger V2d-12 voneinander entkoppelt werden, muß die aufgebrachte Grenzkraft so groß sein, daß die Innenkontur V2d-160 des Klammerausstoßers V2d-16 durch die Keilwirkung der Außenkontur V2d-121 so weit radial nach außen gedrückt wird, daß die Innenkontur V2d-160 vollständig aus der Außenkontur V2d-121 austritt.

### Bezugszeichenliste

- 1: Baugruppe Klammermagazin
- 11: Gehäuse einer Kopfeinheit
- 111: Absatz an Innenkontur von 11
- 112: Druckknopfanschluß an 11
- 12: Skalpellträger
- 121: Führungsnut von 12
- 1211: Vertiefung in 121
- 123: Verzahnung an 12
- 124: Absatz an 12
- 13: Skalpell
- 14: Feder
- 15: Kopplungselemente
- 151: Keilförmige Fläche an 15
- 16: Klammerausstoßer
- 161: Stempel an 16
- 162: Fenster in 16
- 17: Klammermagazin
- 171: Absatz an Magazin 17
- 172: Klammerschächte in 17
- 18: Klammer (u-förmig)

- 2: Baugruppe Verbindungsstift
- 21: Stiftkörper von 2
- 211: Umlaufender Absatz an Innenkontur von 21
- 212: geschlitztes Ende von 21
- 213: Umlaufende Nut an 21 (distal)
- 22: Andruckkörperseitige Verzahnung von 2
- 23: Schaftseitige Verzahnung von 2

- 3: Baugruppe Andruckkörper
- 31: Hutkappe
- 311: Zentrierdorn für Bedienelementfeder an 31
- 312: Führungsbohrung für Ende von 2
- 32: Riegelelement
- 321: Nase von 32
- 322: Kleiner Innendurchmesser von 32
- 323: Großer Innendurchmesser von 32
- 33: Klammermatrize
- 331: Klammerumformnute n in 33
- 332: Verzahnung in 33
- 34: Ring (Schneidengegenlager)
- 35: Bedienelementfeder
- 36: Bedienknopf
- 41: Ballig ausgef. Druckteil
- 42: Schaftrohr
- 420: Radialbohrungen in 42
- 43: Anschlußstift der Andruckkörperverstellur
- 44: Andruckkörper-Verstellmechanik
- 45: Klammerungsmechanik

- V1a-12: Skalpellträger
- V1a-13: Skalpellmesser
- V1a-16: Klammerausstoßer der Variante 1a

- V1b-12: Skalpellträger
- V1b-124: Vertiefung an V1b-12
- V1b-16: Klammerausstoßer der Variante 1b
- V1b-19: Lasche an V1b-16

- V2a-10: Grenzkraftsperre (Sollbruchelement)
- V2a-12: Skalpellträger
- V2a-16: Ausstoßer
- V2a-17: Anschlag von V2a-16

- V2b-10: Grenzkraftsperre (Blattfederraste)
- V2b-12: Skalpellträger
- V2b-124: Vertiefung an V2b-12
- V2b-16: Klammerausstoßer

- V2c-10: Kugel
- V2c-111: Feder
- V2c-12: Skalpellträger
- V2c-121: Vertiefung an V2c-12
- V2c-16: Klammerausstoßer

- V2d-12: Skalpellträger
- V2d-121: Außenkonturwelle von V2d-12
- V2d-16: Klammerausstoßer
- V2d-160: Innenkontur-Welligkeit V2d-16

## Patentansprüche

1. Chirurgisches Instrument in Form eines Nahtklammergeräts, das einen Skalpellträger, ein Zirkularskapell, ein Klammermagazin mit Klammern und eine den Klammern zugeordnete Ausstoßvorrichtung und einen Andruckkörper als Funktionselemente aufweist, die mittels einer vom chirurgischen Instrument lösbaren Bedienvorrichtung mit Griff- und Schaftteil betätigbar sind, **dadurch gekennzeichnet, dass** zum lösbaren Anschließen des Instruments am Schaftteil (42) der Bedienvorrichtung am proximalen Ende des Instruments ein Druckknopfanschluß (112) mit an Biegelaschen sitzenden Druckknöpfen ausgebildet ist und am distalen Ende des Schaftteils (42) zum Eingreifen der Druckknöpfe eine zu diesen korrespondierende Aufnahme (420) ausgebildet ist,
dass zum lösbaren Anschließen der Funktionselemente (V1a-12, V1a-16) des Instruments an dessen proximalem Ende ein Verbindungsteil (2) vorgesehen ist, das mit einem am distalen Ende des Schaftteils (42) ausgebildeten Anschluß (43) mittels einer im Schaftteil (42) untergebrachten, von der Bedienungeinrichtung aus betätigten Verstellmechanik (44) verriegelbar ausgebildet ist, und
zum gleichzeitigen Durchführen einer Klammerung und eines Gewebeschnitts die Funktionselemente Skalpellträger (V1a-12) und Klammerausstoßvorrichtung (V1a-16) als fest gekoppelte Einheit axial verschiebbar sind.

2. Instrument nach Anspruch 1, **dadurch gekennzeichnet, dass** die Funktionselemente Skalpellträger (V2a-12) und Klammerausstoßvorrichtung (V2a-16) axial verschiebbar in der Weise gekoppelt sind, daß nach Durchführen einer Klammerung Skalpellträger (V2a -12) und Klammerausstoßvorrichtung (V2a -16) entkoppelt werden und anschließend durch Verfahren des Skalpellträgers (V2a -12) mittels des Skalpells ein Gewebeschnitt durchgeführt wird.

3. Vorrichtung nach Anspruch 2, **dadurch gekennzeichnet, dass** als Kopplungs/Entkopplungselement zwischen Skalpellträger (V2d-12) und Klammerausstoßvorrichtung (V2d-16) ein biegeelastisches Teil (V2d-160) vorgesehen ist.

4. Vorrichtung nach Anspruch 2, **dadurch gekennzeichnet, dass** als Kopplungs/Entkopplungselement eine Grenzkraftsperre (V2a-10, V2b-10, V2c-10, V2d-160) vorgesehen ist, die bei Überschreiten der Grenzkraft Skalpellträger (V2a-12, V2b-12, V2c-12, V2d-12) und Klammerausstoßvorrichtung (V2a-16, V2b-16, V2c-16, V2d-16) entkoppelt.

5. Vorrichtung nach Anspruch 4, **dadurch gekennzeichnet, dass** die Grenzkraftsperre als Sollbruchelement (V2a-10) ausgebildet ist.

6. Vorrichtung nach Anspruch, 4, **dadurch gekennzeichnet, dass** die Grenzkraftsperre als Blattfederraste (V2b-10) ausgebildet ist.

7. Vorrichtung nach Anspruch 4, **dadurch gekennzeichnet, dass** die Grenzkraftsperre als Kugelfederraste (V2c-10, V2c-111) ausgeführt ist.

8. Vorrichtung nach Anspruch 4, **dadurch gekennzeichnet, dass** zum Koppeln/Entkoppeln einer wellenförmigen Innenstruktur (V2d-160) an der Klammerausstoßvorrichtung (V2d-16) eine korrespondierende Außenkontur (V2d-121) des Skalpellträgers (V2d-12) mittels Keilwirkung zugeordnet ist.

9. Vorrichtung nach Anspruch 1, **dadurch gekennzeichnet, dass** das Verbindungsteil (2), das ein rohrförmiger, am proximalen Ende geschlitzter Stiftkörper (21) ist, Verzahnungsbereiche (22, 23) aufweist, und auf einen im Schaftteil (42) untergebrachten Anschluß (43) der Verstellmechanik (44) aufsteckund verriegelbar ist.

## Claims

1. A surgical instrument in the form of a suture stapler comprising a scalpel holder, a circular scalpel, a staple cartridge including staples, and a staple ejector assigned to said staples and a clincher as function elements actuatable by means of an operating control device including a handle part and a shank part, releasable from said surgical instrument, **characterized in that** for releasably connecting said instrument by said shank part (42) of said operating control device a push-button fastener (112) including push-buttons located on bending tabs is configured at the proximal end of said instrument and a receiving part (420) correspondingly configured for receiving said push-buttons is configured at the distal end of said shank part (42), and that for releasably connecting said instrument function elements (V1a-12, V1a-16) a mandrel assembly (2) is provided at the proximal end of the instrument, said mandrel assembly (2) being configured latchable with a connector (43) at the distal end of said shank part (42) by means of an adjuster mechanism (44) incorporated in said shank part (42) actuatable by said operating control means, and that for simultaneously implementing a stapling action and excision said scalpel holder (V1a-12) and said staple ejector (V1a-16) are axially shiftable as a fixedly coupled assembly.

2. The instrument as set forth in claim 1, **characterized in that** said scalpel holder (V2a-12) and staple ejector (V2a-16) function elements are coupled axially shiftable in such a way that following implementation of a stapling action said scalpel holder (V2a-12) and said staple ejector (V2a-16) are decoupled and subsequently said scalpel holder (V2a-12) is travelled so that by means of said scalpel an excision is implemented.

3. The apparatus as set forth in claim 2, **characterized in that** a flexible part (V2d-160) is provided as coupling and decoupling element between scalpel holder (V2d-12) and staple ejector (V2d-16).

4. The apparatus as set forth in claim 2, **characterized in that** a breakaway lock (V2a-10, V2b-10, V2c-10, V2d-160) is provided as coupling and decoupling element, said breakaway lock decoupling said scalpel holder (V2a-12, V2b-12, V2c-12, V2d-12)) and said staple ejector (V2a-16, V2b-16, V2c-16, V2d-16) on the breakaway force being exceeded.

5. The apparatus as set forth in claim 4, **characterized in that** said breakaway lock is configured as a frangible knockout (V2a-10).

6. The apparatus as set forth in claim 4, **characterized in that** said breakaway lock is configured as a leaf spring latch (V2b-10).

7. The apparatus as set forth in claim 4, **characterized in that** said breakaway lock is configured as a ball spring latch (V2c-10, V2c-111).

8. The apparatus as set forth in claim 4, **characterized in that** for coupling and decoupling, a corrugated inner structure (V2d-160) is assigned a corresponding outer contour (V2d-121) of said scalpel holder (V2d-12) at said staple ejector (V2d-16) by means of a keying effect.

9. The apparatus as set forth in claim 1, **characterized in that** said mandrel assembly (2) is a tubular pin body (21) slotted at the proximal end with spline portions (22, 23) and is mountable on and latchable to a contact pin (43) incorporated in said shank part (42) of said adjuster mechanism (44).

## Revendications

1. Instrument chirurgical sous la forme d'une agrafeuse qui comprend à titre d'éléments fonctionnels un porte-scalpel, un scalpel circulaire, un magasin à agrafes avec des agrafes et un dispositif d'éjection associé aux agrafes et un corps de pressage, qui sont actionnables au moyen d'un dispositif de manipulation détachable de l'instrument chirurgical et comportant une partie de poignée et une partie de tronc, **caractérisé en ce que** pour le raccordement détachable de l'instrument à la partie de tronc (42) du dispositif de manipulation, un raccord à boutons-poussoirs (112) est réalisé à l'extrémité proximale de l'instrument et est pourvu de boutons-poussoirs reposant sur des pattes flexibles, et un logement (420) correspondant aux boutons-poussoirs est réalisé à l'extrémité distale de la partie de tronc (42) pour l'engagement desdits boutons, **en ce que** pour le raccordement détachable des éléments fonctionnels (V1a-12, V1a-16) de l'instrument, il est prévu à son extrémité proximale une pièce de jonction (2) qui est réalisée de manière à pouvoir être verrouillée avec un raccord (43), réalisé à l'extrémité distale de la partie de tronc (42), au moyen d'un mécanisme de déplacement (44) logé dans la partie de tronc (42) et actionné à partir du dispositif de manipulation, et **en ce que** pour effectuer à la fois un agrafage et une coupe tissulaire, les éléments fonctionnels que sont le porte-scalpel (V1a-12) et le dispositif d'éjection d'agrafe (V1a-16) sont axialement déplaçables sous forme d'une unité solidairement couplée.

2. Instrument selon la revendication 1, **caractérisé en ce que** les éléments fonctionnels que sont le porte-scalpel (V2a-12) et le dispositif d'éjection d'agrafe (V2a-16) sont axialement déplaçables en étant couplés de telle sorte qu'après exécution d'un agrafage, le porte-scalpel (V2a-12) et le dispositif d'éjection d'agrafe (V2a-16) sont découplés, et ensuite une coupe tissulaire est effectuée au moyen du scalpel par déplacement du porte-scalpel (V2a-12).

3. Dispositif selon la revendication 2, **caractérisé en ce qu'**il est prévu une pièce élastiquement flexible (V2d-160) à titre d'élément de couplage/découplage entre le porte-scalpel (V2d-12) et le dispositif d'éjection d'agrafe (V2d-16).

4. Dispositif selon la revendication 2, **caractérisé en ce qu'**il est prévu à titre d'élément de couplage/découplage un organe de blocage à force limite (V2a-10, V2b-10, V2c-10, V2d-160) qui, en cas de dépassement de la force limite découple le porte-scalpel (V2a-12, V2b-12, V2c-12, V2d-12) et le dispositif d'éjection d'agrafe (V2a-16, V2b-16, V2c-16, V2d-16).

5. Dispositif selon la revendication 4, **caractérisé en ce que** l'organe de blocage à force limite est réalisé sous forme d'élément destiné à la rupture (V2a-10).

6. Dispositif selon la revendication 4, **caractérisé en ce que** l'organe de blocage à force limite est réalisé sous forme de cran à lame de ressort (V2b-10).

7. Dispositif selon la revendication 4, **caractérisé en ce que** l'organe de blocage à force limite est réalisé sous forme de cran à bille chargée par ressort (V2c-10, V2c-111).

8. Dispositif selon la revendication 4, **caractérisé en ce que** pour le couplage/découplage, il est prévu une structure intérieure ondulée (V2d-160) sur le dispositif d'éjection d'agrafe (V2d-16), à laquelle est associé un contour extérieur correspondant (V2d-121) du porte-scalpel (V2d-12) avec effet de coincement.

9. Dispositif selon la revendication 1, **caractérisé en ce que** la pièce de jonction (2) qui est un corps de tige (21) tubulaire et fendu à l'extrémité proximale comprend des zones dentées (22, 23) et est susceptible d'être enfichée et verrouillée sur un raccord (43), logé dans la partie de tronc (42), du mécanisme de déplacement (44).
